# EUROPÄISCHE PATENTSCHRIFT

(11) **EP 3 025 749 B1**
(45) Veröffentlichungstag und Bekanntmachung des Hinweises auf die Patenterteilung: **23.10.2019**
(21) Anmeldenummer: 15195172.0
(22) Anmeldetag: 18.11.2015
(51) Int. Cl.: A61M 16/04, A61M 29/00

(54) **DILATATOR FÜR PERKUTANE DILATATIONS-TRACHEOTOMIE**
DILATOR FOR PERCUTANEOUS DILATION TRACHEOTOMY
DILATATEUR POUR TRACHÉOTOMIE PERCUTANÉE PAR DILATATION

(30) Priorität: 26.11.2014 DE 102014117368
(43) Veröffentlichungstag der Anmeldung: 01.06.2016
(73) Patentinhaber: Tracoe Medical GmbH, 55268 Nieder-Olm (DE)
(72) Erfinder: Schnell, Ralf, 63500 Seligenstadt (DE)
(74) Vertreter: WSL Patentanwälte Partnerschaft mbB

(56) Entgegenhaltungen:
- US-A1- 2013 255 694
- US-A1- 2014 083 432

## Beschreibung

Die vorliegende Erfindung betrifft einen Dilatator zum Erweitern eines Tracheostomas, der rohrförmig ist und einen Griffbereich und einen sich erweiternden Dilatationsbereich aufweist, wobei zumindest ein Teilabschnitt des Dilatationsbereichs eine Biegung aufweist, so dass der Dilatator eine radial außen- und eine radial innenliegenden Seite aufweist.

Für das Einbringen einer Tracheostomiekanüle in einen Patienten ist die perkutan dilatative Methode nach Ciaglia u.a. aus der US 2004/0098013 A1 bekannt, bei welcher zunächst durch eine Nadel bzw. mit Hilfe eines Skalpells eine sehr kleine Öffnung zu schaffen, die sich durch den Hals bis in die Trachea erstreckt. Durch diese Nadel wird dann ein Führungsdraht, auch Seldinger-Draht genannt, eingeführt. Nach Entfernen der Nadel wird ein Führungskatheter über den Führungsdraht geschoben, über welchen wiederum sukzessive ein oder mehrere konische, gebogene Dilatatoren geschoben und durch die zunächst sehr kleine Öffnung gedrückt werden, wodurch diese erweitert wird. Eine Erweiterung erfolgt in der Regel bis zu einem Innendurchmesser der Öffnung von 13,7 mm (41 French), der dem Außendurchmesser des Dilatators an dieser axialen Position entspricht.

Zur Erleichterung der Einführung des Dilatators sind zahlreiche unterschiedliche Formen und auch strukturierte Dilatatoren im Stand der Technik beschrieben, z. B. in der EP 1 415 679 und der WO 2007/00159. Ungeachtet der Verbesserungen, die diese Systeme teilweise in Bezug auf das Gleiten eines Dilatators in eine zu dehnende Öffnung bereitstellen, besteht bei der Einführung grundsätzlich das Problem, dass während der Dilatation eine im Wesentlichen punktuelle Belastung auf die Mitte der Knorpelspangen ausgeübt wird, zwischen die der Dilatator geschoben wird, und diese in ca. 10 bis 30% der Fälle bei der Dilatation brechen. Derartige gebrochene Knorpelspangen können Stenosen und andere Komplikationen auslösen.

In der Regel erfolgt ein solcher Knorpelspangenbruch an der Knorpelspange, die oberhalb der zu erweiternden Öffnung liegt, d.h. bei der Knorpelspange, die sich näher in Richtung des Kopfes des Patienten findet. Ein Grund dafür ist, dass der Anwender eines Dilatators diesen mit Hilfe einer Kippbewegung in Richtung Kopf durch die zu erweiternde Öffnung führt, um sicherzustellen, dass der gebogene Dilatator dem gebogenen Verlauf der Trachea folgt. Aufgrund dieser Kippbewegung wird auf die obere Knorpelspange mehr Druck ausgeübt als auf die zum Körper gerichtete untere Knorpelspange.

Die Druckschrift US 2013/255694 A1 betrifft eine medizinische Vorrichtung zum Durchführen einer perkutanen Dilatationstracheotomie mit einem Dilatator, der ein ungleichmäßiges Oberflächenprofil aufweist. Das ungleiche Oberflächenprofil ist an die jeweilige Anatomie angepasst, um eine reduzierte, gleichmäßige von dem Dilatator bewirkte Kraft bei der Durchführung der Dilatationstracheotomie bereitzustellen

Vor diesem Hintergrund bestand die Aufgabe, einen Dilatator bereitzustellen, mit dem ein mittiges Einführen in die Trachea möglich ist, der jedoch das Risiko eines Bruchs der oberen Knorpelspange und gegebenenfalls auch der unteren Knorpelspange verringert.

Erfindungsgemäß wird diese Aufgabe gelöst durch einen Dilatator der eingangs genannten Art, bei dem in wenigstens einem Unterabschnitt des Dilatationsbereichs der Außenquerschnitt des Dilatators im Vergleich zu einem runden Querschnitt wenigstens an der radial außen liegenden Seite des Dilatators abgeflacht ist, wobei der Dilatator in wenigstens einem Unterabschnitt des Dilatationsbereichs an der radial außenliegenden Seite des Dilatators eine Rille aufweist, die in Längsrichtung des Dilatators verläuft, und in dem Unterabschnitt, in dem die Rille an der radial außenliegenden Seite des Dilatators in Längsrichtung verläuft, die Abflachung im Außenquerschnitt durch eine Gerade definiert ist und die Rille zentral innerhalb der Abflachung verläuft.

Eine derartige Abflachung an der radial außen liegenden Seite bewirkt, dass bei Einführen des Dilatators bei einer Kippbewegung nach oben während der Durchführung des Dilatators im Bereich der Knorpelspangen der Trachea keine punktuelle Kraftübertragung erfolgt, sondern die Kraft auf eine größere Fläche verteilt wird. Dadurch wird das Risiko eines Bruchs der Knorpelspange oberhalb des Tracheostomas verringert.

Ein solcher Dilatator ist für eine perkutan dilatative Einbringung einer Tracheaostomiekanüle geeignet, bei welcher zunächst durch eine Nadel bzw. mit Hilfe eines Skalpells eine sehr kleine Öffnung angelegt wird, die sich unterhalb der Kehlkopfs durch den Hals bis in die Trachea erstreckt. Durch diese Nadel wird dann ein Führungsdraht, auch Seldinger-Draht genannt, eingeführt. Nach Entfernen der Nadel wird optional ein Führungskatheter über den Führungsdraht geschoben und unmittelbar über den Führungsdraht bzw. über den Führungskathether kann dann der erfindungsgemäße Dilatator geschoben und in die vorbereitete Öffnung gedrückt werden, die bei weiterem Einbringen des Dilatators sukzessive geweitet wird.

Der Begriff "runder Querschnitt" umfasst dabei sowohl kreisrunde als auch leicht elliptische und leicht ovale Formen, wobei sich bei den beiden zuletzt genannten in bestimmten Ausführungsformen der größte und der kleinste Durchmesser des Außenquerschnitts um einen Faktor von höchstens 1,001 bis 1,03 unterscheiden. Derartige Abweichungen beruhen im Wesentlichen auf produktionsbedingten Toleranzen. Vorzugsweise wird jedoch von einem kreisrunden Querschnitt ausgegangen.

Es versteht sich, dass die Abflachung in dem gesamten wenigstens einen Unterabschnitt vorhanden ist, d.h. dass an jeder axialen Position innerhalb des Unterabschnitts bzw. über die gesamte Längserstreckung des wenigstens einen Unterabschnitts der Außenquerschnitt des Dilatators an der radial außen liegenden Seite abgeflacht ist. Vorzugsweise ist die Ausgestaltung der Abflachung des Außenquerschnitts des Dilatators innerhalb eines Unterabschnitts hinsichtlich Form, Ausdehnung, d.h. Winkelbereich, über den sich die Abflachung erstreckt, und Ausprägung, d.h. Maß der Abweichung von einem runden Querschnitt, im Wesentlichen an jeder axialen Position konstant, wobei ggf. produktionsbedingte leichte Abweichungen vorhanden sind.

Die hierin verwendeten Begriffe "distal" und "proximal" beziehen sich auf den Benutzer des Dilatators, d.h. dass sich der Griffbereich am proximalen Ende des Dilatators befindet. Der Dilatationsbereich erweitert sich vom distalen Ende des Dilatators und damit des Dilatationsbereichs zum proximalen Ende des Dilatationsbereichs. Das proximale Ende des Dilatationsbereichs ist das Ende, an das sich der Griffbereich anschließt.

Vorzugsweise beträgt der Außendurchmesser des Dilatators am distalen Ende von 2 mm bis 5 mm, wobei sich diese Maße auf den größten Außendurchmesser an dieser axialen Position beziehen.

Vorzugsweise beträgt die axiale Länge des Dilatationsbereichs von 50 mm bis 140 mm.

Vorzugsweise beträgt die axiale Länge des Griffbereichs von 70 mm bis 100 mm.

Vorzugsweise weist der Dilatator im Dilatationsbereich eine Biegung auf, deren Radius im Bereich von 60 mm bis 120 mm, besonders bevorzugt 75 mm bis 105 mm beträgt. Es handelt sich dabei um den Biegungsradius der zentralen Achse des Dilatators.

Bevorzugte Materialien, aus denen der Dilatator besteht weisen eine Shore Härte von Shore A 80 bis Shore D 70 auf. Die Shore-Härte wird durch dem Fachmann bekannte Verfahren bei einer Temperatur von 23 °C gemessen. Die Messung erfolgt gemäß DIN EN ISO 868 bzw. DIN ISO 7619-1. Derartige Materialien sind formstabil, lassen jedoch ein leichtes Verbiegen zu.

Bevorzugte Materialien umfassen Polyurethan, Weich-PVC, SEBS, Silikon, Polypropylen, Polyethylen und Polyether-Block-Amid-Block-Copolymere.

In einer Ausführungsform erfolgt die Abflachung dadurch, dass eine Rohrwandung des rohrförmigen Dilatators im Bereich der Abflachung dünner ist, als in den Bereichen des Umfangs der Rohrwandung, in denen keine Abflachung vorhanden ist.

In einer anderen Ausführungsform ist die Rohrwandung des rohrförmigen Dilatators in dem Bereich der Abflachung gleich stark wie an den übrigen Bereichen des Umfangs der Rohrwandung, sodass ihre innere Form mit der äußeren Form des Dilatators korrespondiert, wobei sich diese Angabe auf eine jeweilige axiale Position eines Dilatators beziehen und nicht ausschließen, dass die Stärke der Rohrwandung des gesamten Dilatators über dessen gesamte Längserstreckung und insbesondere innerhalb des Dilatationsbereichs variiert. Vorzugsweise nimmt die Stärke der Rohrwandung des Dilatators im Dilatationsbereich von dem distalen Ende bis zu dem an den Griffbereich angrenzenden Abschnitt zu.

In einer Ausführungsform weist der Dilatator in seinem Dilatationsbereich axiale Positionen auf, an denen die Wandstärke der Rohwandung im Bereich der Abflachung geringer ist und axiale Positionen, an denen die innere Form mit der äußeren korrespondiert. Die Gestaltung der inneren Form ist dabei unabhängig von den durch die äußere Form definierten Unterabschnitten.

Die Begriffe "abgeflacht" bzw. "Abflachung" beziehen sich darauf, dass in diesem Unterabschnitt des Umfangs des rohrförmigen Dilatators die Außenkontur des Querschnitts durch eine Gerade definiert wird oder zumindest durch eine Krümmung mit einem oder mehreren größeren Krümmungsradien, als der Krümmungsradius eines minimalen Kreises, in den die gesamte Außenkontur einbeschrieben werden kann. Vorzugsweise ist die Außenkontur des Querschnitts in den Abschnitten, in denen keine Abflachung vorhanden ist, rund bzw. weist im Wesentlichen den gleichen Krümmungsradius auf.

Die Begriffe "Außenquerschnitt" und "Außenkontur des Querschnitts" werden hierin synonym verwendet. Der Querschnitt bezieht sich dabei auf einen Schnitt durch den Dilatator, der senkrecht zu einer in dem rohrförmigen Dilatator verlaufenden zentralen Achse verläuft. Die in dem rohrförmigen Dilatator verlaufende zentrale Achse ist dabei ungeachtet von etwaigen Abflachungen so angeordnet, dass sie durch den Mittelpunkt eines minimalen Kreises verläuft, in den die gesamte Außenkontur an der jeweiligen axialen Position einbeschrieben werden kann.

Die Begriffe "Außendurchmesser" und "Durchmesser de Außenkontur" werden hierin synonym verwendet.

Der unbestimmte Artikel "ein" bzw. "eine", wie er hierin verwendet wird, allgemein genau 1 sowie mehrere umfasst. Dies trifft insbesondere auf die Verwendung im Zusammenhang mit Unterabschnitten des Dilatationsbereichs zu.

Es versteht sich, dass der wenigstens eine Unterabschnitt sich in einer Ausführungsform über einen Teil des Dilatationsbereichs erstreckt, vorzugsweise über einen Teil oder den gesamten Teilabschnitt des Dilatationsbereichs, der eine Biegung aufweist, während sich der wenigstens eine Unterabschnitt in einer anderen Ausführungsform über den gesamten Dilatationsbereich einschließlich eines eventuell nicht gebogenen Teilabschnitts erstreckt.

In einer Ausführungsform erstreckt sich der wenigstens eine Unterabschnitt des Dilatationsbereichs entlang wenigstens 50 % und maximal 100 % des Dilatationsbereichs.

In einer Ausführungsform weist der gesamte Dilatationsbereich eine Biegung auf, d.h. der Teilabschnitt, der eine Biegung aufweist, erstreckt sich über den gesamten Dilatationsbereich, während in einer anderen Ausführungsform nur ein Teilabschnitt des Dilatationsbereich gebogen ist, wobei dieser Teilabschnitt zumindest einen mittleren Abschnitt des Dilatationsbereichs umfasst. Das heißt, dass in einer Ausführungsform der sich an den Griffbereich anschließende Abschnitt und/oder der von dem Griffbereich abgewandte distale Endabschnitt des Dilatationsbereichs gerade verlaufen.

Bei einem Dilatator gehen der Griffbereich und der sich erweiternde Bereich im Wesentlichen nahtlos ineinander über, wobei der Griffbereich einen konstanten Durchmesser haben kann, die Erweiterung des sich erweiternden Dilatationsbereichs fortsetzen kann oder seinerseits eine Erweiterung in die entgegengesetzte Richtung aufweisen kann.

In der Regel ist die Abgrenzung zwischen Griffbereich und sich erweiterndem Dilatationsbereich bei einem Dilatator durch eine Markierung an der Außenseite des rohrförmigen Dilatators gekennzeichnet, die angibt, bis zu welcher axialen Position der Dilatator in das zu erweiternde Tracheostoma eingeführt werden kann und darf, um eine vordefinierte Tracheostomagröße zu erzeugen. Eine übliche Größe bzw. Weite eines Tracheostomas beträgt 13,7 mm, d.h. 41 French. Das bedeutet, dass der Außendurchmesser des Dilatators an der axialen Position der Markierung, d.h. der axialen Position der Abgrenzung zwischen Griffbereich und Dilatationsbereich 13,7 mm beträgt, wobei hier von einem kreisrunden Querschnitt ausgegangen wird, bzw. von einem Durchmesser eines minimalen Kreises, in den die Außenkontur des Querschnitts des Dilatators eingeschrieben werden kann.

In bestimmten Ausführungsformen beträgt bei einem erfindungsgemäßen Dilatator an einer axialen Position des Dilatationsbereichs der größte Außendurchmesser gemessen von der radial innen liegenden Seite zur radial außen liegenden Seite im Bereich von 12 mm bis 13,5 mm, während der größte Außendurchmesser an einer anderen Position des Dilatationsbereichs, gemessen senkrecht zu der Richtung von radial außen liegender Seite zu radial innen liegender Seite größer als 13,7 mm ist. In einer Ausführungsform beträgt der größte Außendurchmesser des Dilatationsbereichs des Dilatators in der senkrecht zu der Richtung von radial außen liegender Seite zu radial innen liegender Seite liegenden Richtung zwischen 13,8 mm und 14,5 mm.

In einer Ausführungsform ist die Außenkontur des Querschnitts des Dilatators in wenigstens einem Unterabschnitt des Dilatationsbereichs, in dem der Außenquerschnitt des Dilatators im Vergleich zu einem runden Querschnitt wenigstens an der radial außen liegenden Seite des Dilatators abgeflacht ist, zusätzlich an der radial innen liegenden Seite des Dilatators abgeflacht. Durch die Abflachung sowohl an der radial außen liegenden Seite als auch der radial innen liegenden Seite des Dilatators ist bei Einführung des Dilatators der Durchmesser des Dilatators senkrecht zu den Knorpelspangen geringer als parallel zu den Knorpelstangen. Dadurch wird die Wirkung verstärkt, dass eine parallel zu den Knorpelspangen verlaufende Dehnung stärker ausgeprägt ist als eine senkrecht zu den Knorpelspangen verlaufende Dehnung und dadurch ein geringeres Risiko eines Bruchs der Knorpelspangen vorhanden ist. Bei dieser Ausführungsform trifft dies nicht nur für die oberhalb des Tracheostomas verlaufende Knorpelspange, sondern auch für die unterhalb des Tracheostomas verlaufende Knorpelspange zu.

In einer Ausführungsform unterscheiden sich der eine Unterabschnitt und ein oder mehrere weitere Unterabschnitte darin voneinander, dass die radial innen liegende Seite des Dilatators in diesem Unterabschnitt abgeflacht bzw. nicht abgeflacht ist.

In einer Ausführungsform ist die Außenkontur des Querschnitts des Dilatators in dem wenigstens einen Unterabschnitt in einem Kreissektor des Querschnitts abgeflacht, der sich über einen Winkelbereich von 20° bis 100°, vorzugsweise 30° bis 60° erstreckt, bezogen auf einen minimalen Kreis, in den die Außenkontur des Querschnitts an der jeweiligen axialen Position einbeschrieben werden kann.

Diese Angaben zum Winkelbereich beziehen sich in einer Ausführungsform nur auf eine Abflachung an der radial außen liegenden Seite. In einer anderen Ausführungsform beziehen sich diese Angaben auch auf eine Abflachung an der radial innen liegenden Seite des Dilatators.

In einer Ausführungsform ist in dem wenigstens einen Unterabschnitt ein Querschnittsdurchmesser der Außenkontur, gemessen von der radial innen liegenden zu der radial außen liegenden Seite des Dilatators um 3,5 % bis 30 %, vorzugsweise 5 % bis 20 % geringer als ein Querschnittsdurchmesser senkrecht dazu. Die Prozentangabe bezieht sich dabei auf den maximalen Querschnittsdurchmesser der Außenkontur, der als 100 % definiert wird. Bei dieser Ausführungsform ist gewährleistet, dass bei Einführung des Dilatators ein größerer Druck und eine größere Dehnung des Gewebes in seitliche Richtung erfolgt, als in senkrecht zu den Knorpelspangen liegende Richtung.

Erfindungsgemäß weist der Dilatator in wenigstens einem Unterabschnitt an der radial außen liegenden Seite des Dilatators eine Rille auf, die in Längsrichtung des Dilatators verläuft. Eine derart verlaufende Rille befindet sich im Wesentlich im Bereich der Abflachung des wenigsten einen Unterabschnittes.

Der Begriff "eine Rille" bezeichnet hierin genau eine Rille.

Bei derartigen Ausführungsformen mit Rille wird bei Einführung des Dilatators der Druck auf mehrere Bereiche der oberen Knorpelspangen verteilt und dadurch die Gefahr eines Knorpelbruches zusätzlich verringert.

Die Rille weist in einer Ausführungsform in einem Querschnitt eine einseitig offene runde Form, insbesondere einen einseitig offenen Halbkreis, oder eine vier- oder mehreckige Form auf, die nach einer Seite offen ist.

In einer bevorzugten Ausführungsform ist die Rille in dem wenigstens einen Unterabschnitt lediglich an der radial außen liegenden Seite des Dilatators vorhanden. Zusätzlich erfolgt in einer Ausführungsform eine weitere Abflachung an der radial innen liegenden Seite des Dilatators, wobei diese vorzugsweise keine zusätzliche Rille oder Nut aufweist.

In Ausführungsformen, bei denen eine Rille vorhanden ist, kann ein Querschnittsdurchmesser der Außenkontur, gemessen von der radial innen liegenden zu der radial außen liegenden Seite des Dilatators um 3,5 bis 30%, vorzugsweise 5 % bis 20 % geringer sein als ein Querschnittsdurchmesser senkrecht dazu. Der Außendurchmesser gemessen von der radial innen liegenden zu der radial außen liegenden Seite des Dilatators wird in solchen Fällen bei entsprechender Position der Rille vom tiefsten Punkt der Rille gemessen.

In einer Ausführungsform weist der Außenquerschnitt an den axialen Positionen, an denen die Rille vorhanden ist, an Stelle einer die Abflachung definierenden Geraden eine konkave Einwölbung oder Einbuchtung auf, d.h. die Rille ersetzt die Abflachung bei dieser Ausführungsform.

In einer Ausführungsform weist die Rille eine Tiefe im Bereich von 0,05 mm bis 2,5 mm auf, vorzugsweise im Bereich von 0,3 mm bis 1,5 mm. Die Tiefe wird zwischen der größten Tiefe der Rille und einer gedachten Fortsetzung der runden Form des Außenquerschnitts des Dilatators gemessen. Durch eine derartige Tiefe wird bewirkt, dass die Kraft, die der Benutzer bei Einführung des Dilatators und Aufweitung des Tracheostomas auf die obere Knorpelspange ausübt auf zwei beabstandete Bereiche verteilt wird, wobei andererseits keine zu tiefe Rille erzeugt wird, die den Dilatator instabil machen würde.

In einer Ausführungsform beträgt die Breite der Rille im Bereich von 1 mm bis 10 mm, und /oder nimmt proportional zum Querschnittsdurchmesser der Außenkontur des Dilatators, gemessen senkrecht zu der Richtung von radial außen liegender zu radial innen liegender Seite, zu. Durch eine derartige Variation der Breite der Rille kann sichergestellt werden, dass die Kraft, die durch unterschiedliche axialen Positionen des Dilatators auf die Knorpelspangen ausgeübt wird, an jeder Position optimal verteilt wird. Dazu ist es erforderlich, dass die Rillenbreite näher an dem Endabschnitt des Dilatators geringer ist als an einer axialen Position, die sich näher in Richtung des Griffbereichs befindet.

In einer Ausführungsform erstreckt sich die Rille über einen Bereich, der 50 % des Dilatationsbereichs beträgt und maximal 100 % des Dilatationsbereichs, bezogen auf die Längserstreckung des Dilatationsbereichs.

Erfindungsgemäß ist in dem Unterabschnitt, in dem die Rille an der radial außenliegenden Seite des Dilatators in Längsrichtung verläuft, die Abflachung im Außenquerschnitt durch eine Gerade definiert und die Rille verläuft zentral innerhalb der Abflachung.

Vorzugsweise sind zu beiden Seiten der Rille im Außenquerschnitt Abschnitte der durch eine Gerade definierten Abflachung vorhanden.

Bei einer derartigen Ausführungsform sind zwei gerade, voneinander beabstandete Bereiche an den jeweiligen axialen Positionen vorhanden, die bei Einführen des Dilatators Druck auf die obere Knorpelspange ausüben und in denen jeweils eine nicht-punktuelle Kraftübertragung auf die obere Knorpelspange erfolgt.

Es versteht sich, dass durch die zentral innerhalb der Abflachung verlaufende Rille an der jeweiligen axialen Position ein Außenquerschnitt des Dilatators vorhanden ist, bei dem an der radial außen liegenden Seite eine Abflachung vorhanden ist, die zentral eine Rille, d.h. im Querschnitt eine einseitig offene runde Form, insbesondere einen einseitig offenen Halbkreis, oder eine vier- oder mehreckige Form aufweist, die nach einer Seite offen ist, wobei die durch eine Gerade definierte Abflachung des Außenquerschnitts zu beiden Seiten der Rille die gleiche Länge aufweist. Die Rille ist demnach zusätzlich zu einer Abflachung vorhanden, die im Außenquerschnitt durch eine Gerade definiert ist und bewirkt eine zusätzliche Abflachung.

Vorzugsweise erstreckt sich bei dieser Ausführungsform die Abflachung, die durch eine Gerade des Außenquerschnitts definiert ist, zu beiden Seiten der Rille im Querschnitt jeweils über einen Winkelbereich des Querschnitts von 5 ° bis 15 °, bezogen auf einen minimalen Kreis, in den die Außenkontur des Querschnitts an der jeweiligen axialen Position einbeschrieben werden kann.

In einer Ausführungsform nimmt in dem sich vom distalen Ende zum Griff hin erweiternden Dilatationsbereich der äußere Umfang des Dilatators kontinuierlich zu. Das distale Ende bezeichnet hierin das von dem Griffbereich abgewandte Ende des Dilatators. Die Abnahme erfolgt in der Regel linear kann jedoch bei bestimmten Ausführungsformen einem nicht linearen aber regelmäßigen Muster folgen.

Dadurch erfolgt durch jede axiale Position des Dilatators eine Dehnung des Tracheostomas, die je nach Ausgestaltung des Dilatators an der jeweiligen axialen Position mehr oder weniger stark parallel oder senkrecht zu den Knorpelspangen ausgeprägt ist.

In einer Ausführungsform weist der Dilatationsbereich mehrere Unterabschnitte auf, in denen der Außenquerschnitt des Dilatators im Vergleich zu einem runden Querschnitt wenigstens an der radial außen liegenden Seite des Dilatators abgeflacht ist, wobei sich wenigstens ein Unterabschnitt der mehreren Unterabschnitte von den jeweils anderen Unterabschnitten des Dilatators dadurch unterscheidet, dass die Abflachung des Außenquerschnitts eine andere Form, Ausdehnung und/oder Ausprägung aufweist.

Es versteht sich, dass auch bei dieser Ausführungsform die Abflachung des Dilatators innerhalb eines Unterabschnitts hinsichtlich Form, Ausdehnung und Ausprägung im Wesentlichen konstant ist. Es versteht sich weiter, dass zwischen den Unterabschnitten Übergangsbereiche vorhanden sind, in denen die Unterabschnitte mit ihren unterschiedlichen Ausgestaltungen hinsichtlich Form, Ausdehnung und/oder Ausprägung ineinander übergehen.

Die Form der Abflachung wird hierin durch den Krümmungsradius des Außenquerschnitts im Bereich der Abflachung, das Vorhandensein einer Geraden beim Außenquerschnitt im Bereich der Abflachung und/oder das Vorhandensein einer Rille im Außenquerschnitt im Bereich der Abflachung definiert.

Die Ausprägung der Abflachung wird hierin durch das Verhältnis des Querschnittsdurchmessers der Außenkontur, gemessen von der radial innen liegenden zu der radial außen liegenden Seite, im Vergleich zu einem Querschnittsdurchmesser senkrecht dazu definiert.

Die Ausdehnung der Abflachung wird hierin durch den Winkelbereich, über den sich die Abflachung erstreckt, definiert.

Durch eine derartige Ausgestaltung ist es möglich, eine größere Dehnung des Tracheostomas entlang der Richtung der Knorpelspangen im Verhältnis zu der Richtung senkrecht zu den Knorpelspangen auszuüben, wobei diese Dehnung durch Bereiche, in denen schwächere Abflachung an der radial außenliegenden Seite und gegebenenfalls an der radial innenliegenden Seite des Dilatators vorhanden ist, nachgeholt wird.

In einer Ausführungsform weisen alle Unterabschnitte einen Querschnittsdurchmesser der Außenkontur, gemessen von der radial innen liegenden zu der radial außen liegenden Seite des Dilatators auf, der um 3,5 % bis 30 %, vorzugsweise 5 % bis 20 %, geringer ist, als ein Querschnittsdurchmesser senkrecht dazu.

In einer Ausführungsform grenzen die mehreren Unterabschnitte, in denen der Außenquerschnitt des Dilatators im Vergleich zu einem runden Querschnitt wenigstens an der radial außen liegenden Seite des Dilatators abgeflacht ist, unmittelbar aneinander an.

In einer Ausführungsform sind genau drei Unterabschnitte vorhanden, in denen der Außenquerschnitt des Dilatators im Vergleich zu einem runden Querschnitt wenigstens an der radial außen liegenden Seite des Dilatators abgeflacht ist, wobei der mittlere der Unterabschnitte ein Verhältnis von Querschnittsdurchmesser der Außenkontur, gemessen von der radial innen liegenden zu der radial außen liegenden Seite des Dilatators, zu Querschnittsdurchmesser senkrecht dazu aufweist, das geringer ist, als in den anderen Unterabschnitten.

In einer Ausführungsform erstreckt sich der wenigstens eine Unterabschnitt, in dem der Außenquerschnitt des Dilatators im Vergleich zu einem runden Querschnitt an der radial außen liegenden Seite des Dilatators abgeflacht ist, über den gesamten Dilatationsbereich. Durch diese Ausführungsform wird gewährleistet, dass innerhalb des gesamten Bereichs, der für die Dehnung des Tracheostomas benutzt wird, eine Druckbelastung der Knorpelspangen an einem einzigen Punkt verhindert wird.

In einer Ausführungsform ist der Dilatator unabhängig von der Auftrennung in Griffbereich und Dilatationsbereich über wenigstens 80 % seiner Längserstreckung, vorzugsweise wenigstens 90 % und bis zu 100 % seiner Längserstreckung gebogen, wobei er vorzugsweise an jeder gebogenen axialen Position den gleichen Biegungsradius aufweist. Es handelt sich dabei um den Biegungsradius der zentralen Achse des Dilatators. Bei einer derartigen Ausführungsform ist für den Anwender leichter erkennbar, wie der Verlauf des bereits in den Patienten eingeführten Bereichs des Dilatators ist.

Vorzugsweise beträgt der Biegungsradius im Bereich von 60 mm bis 120 mm, besonders bevorzugt 75 mm bis 105 mm.

In einer Ausführungsform weist der rohrförmige Dilatator in seinem Lumen einen rohrförmigen Führungskatheter zur Aufnahme eines Führungsdrahtes auf. Bei einer derartigen Ausführungsform ist es nicht erforderlich, einen separaten Führungskatheter in das Lumen des rohrförmigen Dilatators einzuführen.

Vorzugsweise ist der Führungskatheter fest mit dem Dilatator verbunden, besonders bevorzugt durch eine Verklebung. Es ist dabei nicht erforderlich, dass der Führungskathether über den gesamten Verlauf des Dilatators zentral in diesem angeordnet ist, vielmehr ist es ausreichend, wenn der Führungskatheter so gestaltet ist, dass sein Außendurchmesser an dem distalen Ende des Dilatators, das von dem Griffbereich abgewandt ist, dem Innendurchmesser des Dilatators entspricht, so dass an dieser axialen Position automatisch eine Zentrierung erfolgt.

In einer anderen Ausführungsform weist die Öffnung des Dilatators am distalen Ende einen Innendurchmesser von 1,3 bis 1,5 mm auf. Dieser Durchmesser entspricht im Wesentlichen dem Durchmesser eines Seldingerdrahts. Durch eine derartige Ausgestaltung eines Dilatators ist es möglich, bei der Dilatation auf einen Führungskatheter vollständig zu verzichten.

Erfindungsgemäß wird die Aufgabe ebenfalls gelöst durch eine Tracheostomiekanüle mit einem Dilatator gemäß einem der vorangehenden Ansprüche. Dabei ist der Dilatator so ausgestaltet, dass die Tracheostomiekanüle über diesen geführt werden kann und ein Rückziehen des Dilatators durch die Tracheostomiekanüle, wenn diese in das aufgeweitete Tracheostoma eingebracht ist, möglich ist. Spezielle Ausgestaltungen des Dilatators mit elastisch rückklappbaren Schirmen, bei denen in einem ersten Zustand ein Basisdurchmesser des elastisch rückklappbaren Schirms dem Außendurchmesser einer Tracheostomiekanüle entspricht und der in rückgeklapptem Zustand einen Außendurchmesser hat, der geringer ist als der Innendurchmesser der Tracheostomiekanüle, sind dem Fachmann bekannt.

In einer Ausführungsform korrespondiert die äußere Form der Tracheostomiekanüle mit der inneren Form des Dilatators.

In einer Ausführungsform ist der Dilatator mit Hilfe einer Überwurfmutter an der Kanüle arretiert, wobei die Überwurfmutter an dem proximalen Ende der Kanüle bzw. des Dilatators angeordnet ist. Eine solche Arretierung verhindert eine Verrutschen der Kanüle entlang des Dilatators bzw. ein Verrutschen des Dilatators innerhalb der Kanüle. Dadurch wird eine Spaltbildung zwischen distalem Ende der Kanüle und dem Dilatator in diesem Bereich verhindert. Zudem wird bei Ausführungsformen mit einem rückklappbaren Schirm ein unbeabsichtigtes Rückklappen des Schirms unterbunden.

Weitere Vorteile, Merkmale und Anwendungsmöglichkeiten der vorliegenden Erfindung werden deutlich anhand der folgenden Beschreibung bevorzugter Ausführungsformen und den dazugehörigen Figuren.

Es zeigen:
- Figur 1:: eine seitliche Draufsicht auf einen zur Offenbarung gehörenden Dilatators einschließlich verschiedener Querschnitte an unterschiedlichen axialen Positionen,
- Figur 2:: eine seitliche Draufsicht einer weiteren Ausführungsform eines zur Offenbarung gehörenden Dilatators einschließlich Querschnitte an unterschiedlichen axialen Positionen,
- Figur 3:: eine seitliche Draufsicht einer weiteren Ausführungsform eines zur Offenbarung gehörenden Dilatators einschließlich Querschnitte an unterschiedlichen axialen Positionen,
- Figur 4a:: eine seitliche Draufsicht einer weiteren Ausführungsform eines erfindungsgemäßen Dilatators einschließlich Querschnitte an unterschiedlichen axialen Positionen,
- Figur 4b:: eine Vergrößerung des Querschnitts an der axialen Position C-C der Ausführungsform aus Figur 4a,
- Figur 5a:: eine seitliche Draufsicht einer weiteren Ausführungsform eines erfindungsgemäßen Dilatators einschließlich Querschnitte an unterschiedlichen axialen Positionen,
- Figur 5b:: einen Längsschnitt durch die Ausführungsform aus Figur 5a,
- Figur 6:: eine seitliche Draufsicht einer weiteren Ausführungsform eines erfindungsgemäßen Dilatators einschließlich Querschnitte an unterschiedlichen axialen Positionen und
- Figur 7:: eine seitliche Draufsicht einer weiteren Ausführungsform eines erfindungsgemäßen Dilatators einschließlich Querschnitte an unterschiedlichen axialen Positionen.

In den Figuren sind gleiche Elemente mit gleichen Bezugszeichen bezeichnet. Die Figuren sind lediglich schematische Darstellungen, anhand derer das Prinzip der Erfindung erläutert wird, zudem beanspruchen sie keine Maßstabstreue.

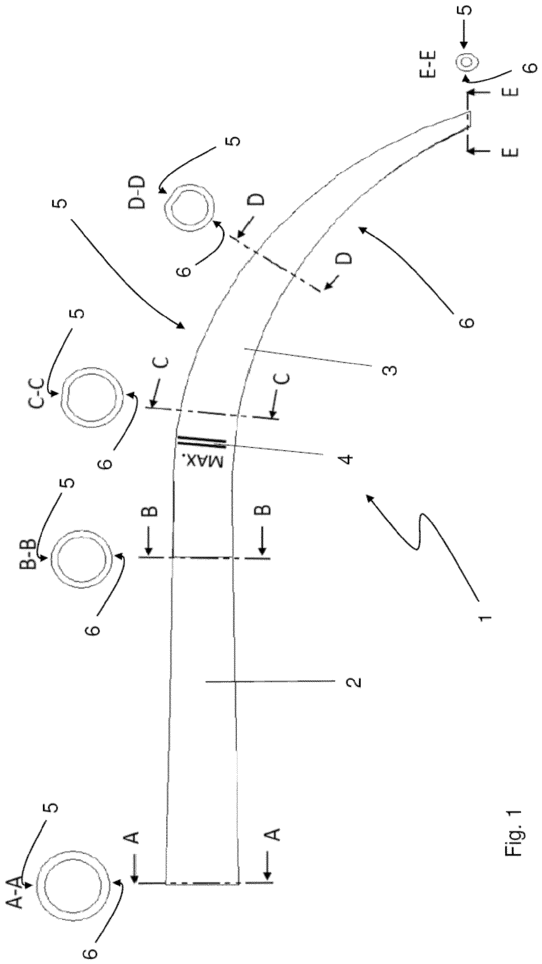

Der in Figur 1 dargestellte Dilatator 1 weist einen Griffbereich 2 sowie einen Dilatationsbereich 3 auf, wobei die Markierung 4 die Grenze zwischen Griffbereich 2 und Dilatationsbereich 1 kennzeichnet. Die Markierung 4 kennzeichnet die axiale Position, bis zu der der Dilatator in ein Stoma maximal eingeführt werden soll. An dieser axialen Position weist der Dilatator einen Durchmesser von 13,7 mm (41 FR) auf. Innerhalb des Dilatationsbereichs können weitere Markierungen vorhanden sein, zur Information bei der Dilatation dienen. Übliche Werte sind bei einem Durchmesser von 12,7 mm (38 FR), wobei sich die angegebenen Durchmesser an der axialen Position der Markierung auf den Durchmesser ohne Abflachung beziehen.

Bei dem in Figur 1 dargestellten Dilatator wird die sich erweiternde Form des Dilatationsbereichs 3 auch im Griffbereich 2 fortgeführt, wobei jedoch die Erweiterung dort weniger ausgeprägt ist. Der Griffbereich 2 erweitert sich von der axialen Position A-A zur axialen Position B-B in Längserstreckung des Dilatators und weiter bis zur axialen Position der Markierung 4.

Anhand der Querschnitte an den axialen Positionen C-C, D-D und E-E, die entlang der gestrichelt gekennzeichneten Linien gemacht wurden, ist deutlich erkennbar, dass der Dilatator 1 an der radial außen liegenden Seite 5 abgeflacht ist. Die radial außen liegende Seite des Dilatators wird definiert durch eine Biegung innerhalb des Dilatationsbereichs 3, wobei sich bei dieser Ausführungsform der Teilabschnitt des Dilatationsbereichs, der eine Biegung aufweist, über den gesamten Dilatationsbereich 3 erstreckt. Weiter ist an den Querschnitten des Dilatators im Dilatationsbereich 3 ebenfalls gut erkennbar, dass lediglich eine Abflachung an der radial außen liegenden Seite 5 des Dilatators vorhanden ist und die radial innen liegende Seite 6 nicht abgeflacht ist.

Zudem ist gut erkennbar, dass bei dieser Ausführungsform an den axialen Positionen C-C und D-D eine Abflachung durch einen geraden Verlauf der äußeren Rohrwandung definiert ist, wobei die gesamte Rohrwandung eine abgeflachte Form aufweist, d.h. dass die Stärke der Rohrwandung im Bereich der Abflachung die gleiche ist wie in den übrigen Bereichen des Umfangs der Rohrwandung an diesen axialen Positionen, sodass die innere Form der Rohrwandung des Dilatators mit der äußeren Form korrespondiert. Lediglich der Querschnitt E-E bildet hierzu eine Ausnahme, da aufgrund des ohnehin relativ geringen Innendurchmessers an dieser axialen Position eine zusätzliche Verengung unter anderem ein Einpassen eines Führungskatheters an dieser Stelle erschweren würde. An der axialen Position des Querschnitts E-E ist daher die Rohrwandung an der radial außen liegenden Seite des Dilatators weniger stark als in den übrigen Bereichen. Der Übergang zwischen den unterschiedlichen Arten der Abflachung erfolgt im Wesentlichen fließend zwischen der axialen Position D-D und E-E.

Insgesamt verringert sich jedoch die Stärke der Rohrwandung vom Ende des Griffbereichs 2 des Dilatators bis zu dem distalen Ende des Dilatators kontinuierlich.

In der in Figur 1 gezeigten Ausführungsform ist genau ein Unterabschnitt des Dilatationsbereichs vorhanden, in dem der Außenquerschnitt des Dilatators an der radial außen liegenden Seite abgeflacht ist.

Bei dieser Ausführungsform ist beispielsweise an der axialen Position C-C eine Abflachung an der Außenkontur des Querschnitts vorhanden, die sich über einen Kreissektor mit einem Winkelbereich von ca. 35° bis 50° erstreckt. Das Gleiche trifft auch für die Querschnitte an den axialen Positionen D-D und E-E zu.

Weiter ist in Figur 1 zudem deutlich erkennbar, dass der Querschnitt des Dilatators im Griffbereich kreisrund ist, sodass dieser Dilatator auf einer im Wesentlichen kreisrunden Form basiert.

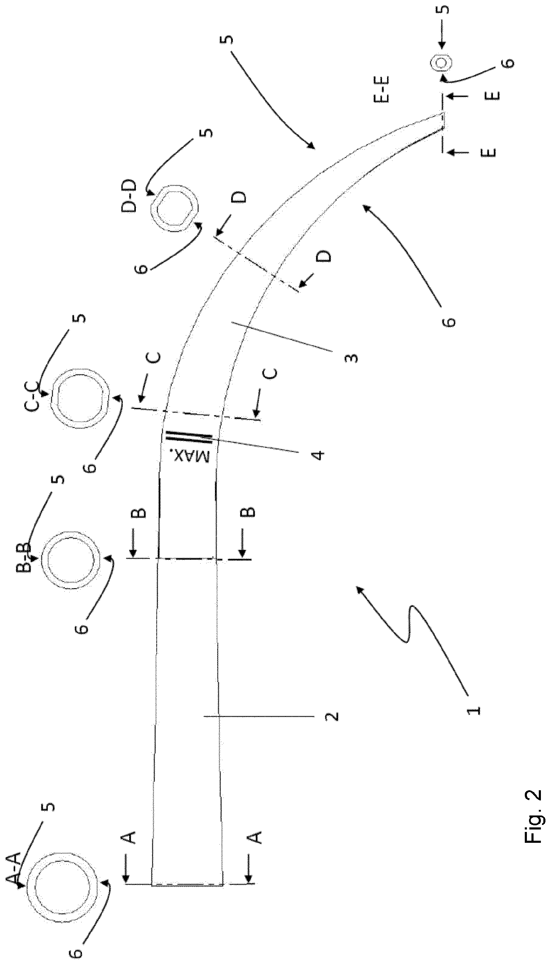

Der in Figur 2 dargestellte Dilatator 1 entspricht im Wesentlichen der in Figur 1 gezeigten Ausführungsform sowohl hinsichtlich der Ausgestaltung des Griffbereichs 2 als auch des Dilatationsbereichs 3. Zusätzlich ist jedoch der Außenquerschnitt in dem wenigstens einen Unterabschnitt, der sich bei dieser Ausführungsform über den gesamten Dilatationsbereich 3 erstreckt, im Vergleich zu einem runden Querschnitt an der radial innen liegenden Seite des Dilatators abgeflacht. Bei dieser Ausführungsform ist die Abflachung der Außenkontur des Querschnitts des Dilatators im gesamten Dilatationsbereich sowohl an der radial außen liegenden Seite als auch an der radial innen liegenden Seite durch eine Gerade definiert.

Auch bei dieser Ausführungsform wird aus den Querschnitten an den axialen Positionen C-C und D-D deutlich, dass die Abflachung durch eine Verformung der gesamten Rohrwandung des Dilatators zustande kommt, während die Abflachung an der axialen Position E-E durch eine dünnere Rohrwandung an der radial außen und radial innen liegenden Seite zustande kommt, um einen runden Innenquerschnitt bereitstellen zu können. Der Übergang zwischen den unterschiedlichen Arten der Abflachung erfolgt im Wesentlichen fließend zwischen der axialen Position D-D und E-E.

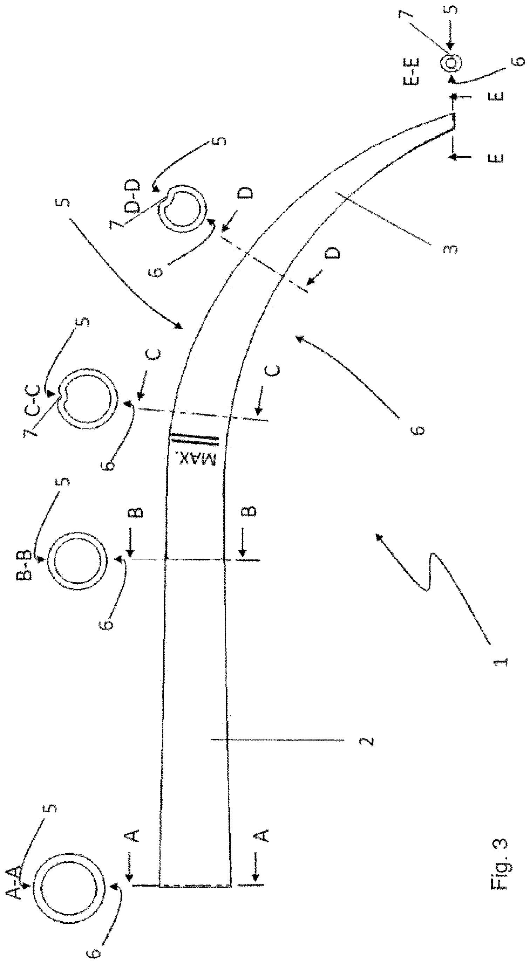

Auch die in Figur 3 dargestellte Ausführungsform entspricht im Wesentlichen der in Figur 1 dargestellten Ausführungsform, wobei anstelle der durch eine Gerade definierten Abflachung des Unterabschnitts des Dilatators, der sich über den gesamten Dilatationsbereich 3 erstreckt, eine Rille 7 vorhanden ist, die im Querschnitt die Form eines einseitig offenen Halbkreises hat.

Wie in Figur 1 ist an den axialen Positionen C-C und D-D des Unterabschnitts bzw. des Dilatationsbereichs 3 die Rohrwandung gleichmäßig stark, einschließlich der axialen Position, an der die Rille 7 vorhanden ist, während an der axialen Position E-E die Rille durch eine Einkerbung in der Rohrwandung zustande kommt und diese an der axialen Position der Rille, durch die die Abflachung definiert wird, eine geringere Stärke aufweist, sodass an der axialen Position E-E der Innenquerschnitt kreisrund ist. Innerhalb des Unterabschnitts, der sich bei dieser Ausführungsform über den gesamten Dilatationsbereich 3 erstreckt, erfolgt der Übergang zwischen den unterschiedlichen Ausgestaltungen der Abflachung durch Einkerbung bzw. Verformung der Rohrwandung fließend. Die Rille, die eine durch eine Gerade definierte Abflachung überdeckt, formt dadurch zwei voneinander beabstandete Bereiche in der Außenkontur des Querschnitts des Dilatators, die Druck auf eine obere Knorpelspange ausüben. Die Rille erstreckt sich im Querschnitt über einen Kreissektorin einem Winkelbereich von 20° bis 40° und somit über einen kleineren Winkelbereich als in Figur 1.

An der radial innen liegenden Seite 6 ist keine Abflachung vorhanden und der Griffbereich 2 weist ebenfalls keine Abflachung auf und ist wie oben für Figur 1 beschrieben ausgestaltet.

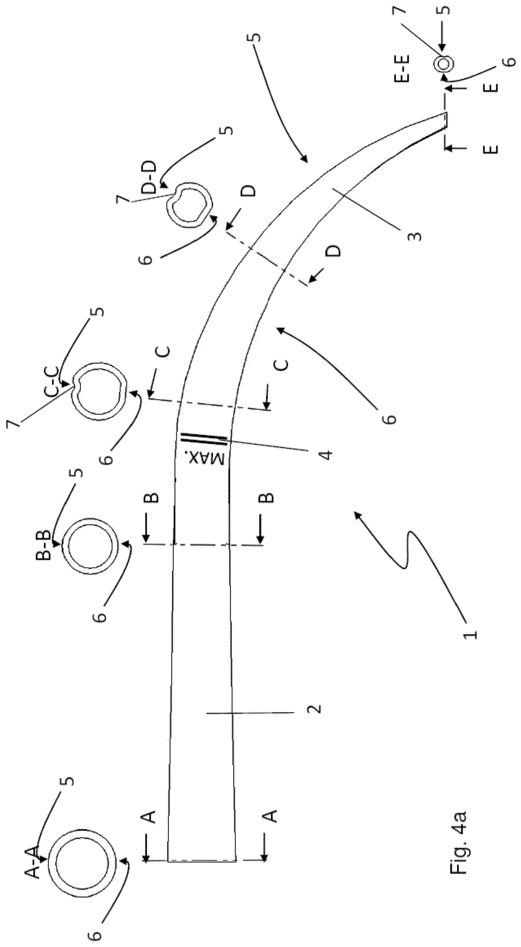

Die in Figur 4a gezeigte Ausführungsform entspricht grundsätzlich im Wesentlichen der in Figur 2 gezeigten Ausführungsform, wobei der Griffbereich 2 an der axialen Position der Markierung 4 in den Dilatationsbereich 3 übergeht, und wobei der Griffbereich 2 ebenfalls eine leichte Erweiterung aufweist, die jedoch im Dilatationsbereich 3 stärker ausgeprägt ist. Zudem weist der rohrförmige Dilatator 1 im Griffbereich sowohl äußerlich als auch innerlich eine kreisrunde Form auf, und der Teilbereich des Dilatationsbereichs, der eine Biegung aufweist, erstreckt sich über den gesamten Dilatationsbereich 3.

Zudem erstreckt sich der Unterabschnitt des Dilatationsbereichs 3, der an der radial außen liegenden Seite eine Abflachung aufweist, ebenfalls über den gesamten Dilatationsbereich 3.

In diesem Bereich weist der Dilatator zusätzlich an der radial innen liegenden Seite 6 eine Abflachung auf.

Die Abflachung an der radial innen liegenden Seite 6 ist durch eine Gerade definiert, während die Abflachung an der radial außen liegenden Seite 5 prinzipiell ebenfalls durch eine Gerade definiert ist, wobei im Bereich der Abflachung zusätzlich eine Rille 7 vorhanden ist, die in der Längsrichtung des Dilatators zentral innerhalb der durch die Gerade definierten Abflachung liegt, sodass die Rille zusätzlich zu der Abflachung an der radial außen liegenden Seite 5 vorhanden ist.

Auch bei dieser Ausführungsform kommen die Abflachungen an den radial außen liegenden und radial innen liegenden Seiten 5, 6 an den axialen Positionen C-C und D-D durch eine Verformung der Rohrwandung des Dilatators zustande, während an der axialen Position E-E die Rohrwandung an der radial außen liegenden Seite und radial innen liegenden Seite weniger stark ist, als in den übrigen Umfangsbereichen an dieser axialen Position, wobei der Innenquerschnitt des Dilatators an dieser axialen Position kreisrund ist.

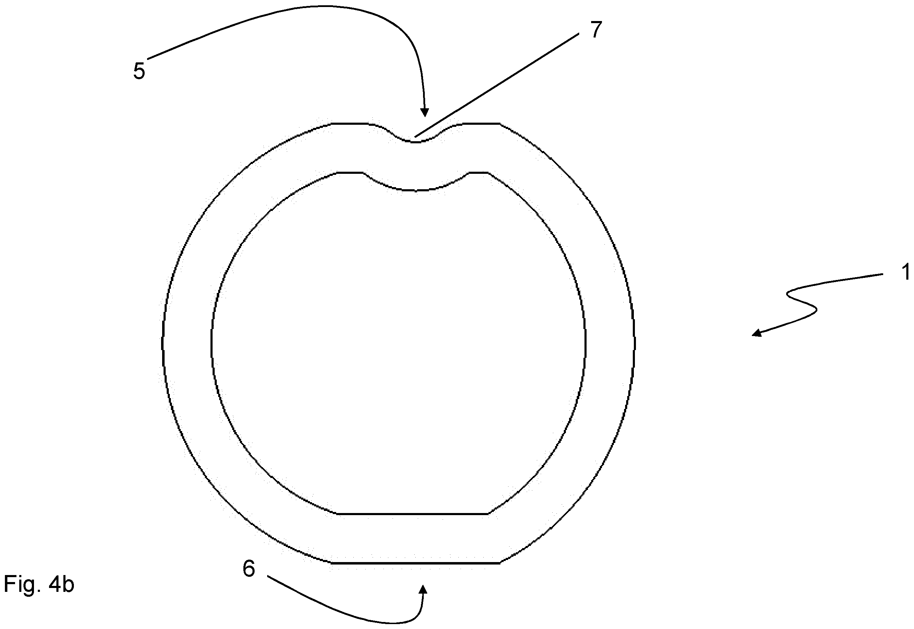

Die Art der Abflachung, insbesondere an der radial außen liegenden Seite, geht noch deutlicher aus der Abbildung in Figur 4b hervor, die den Querschnitt an der axialen Position C-C stark vergrößert darstellt. Bei dieser Abbildung ist deutlich erkennbar, dass an der radial innen liegenden Seite 6 die Abflachung ausschließlich durch eine Gerade definiert wird, während an der radial außen liegenden Seite die Abflachung durch eine Gerade und eine Rille 7 definiert wird.

Bei dieser Ausführungsform ist sowohl an der radial außen liegenden Seite 5 als auch an der radial innen liegenden Seite 6 die Außenkontur des Querschnitts des Dilatators in einem Kreissektor abgeflacht, der sich über einen Winkelbereich von ca. 35 bis ca. 45° erstreckt, wobei selbstverständlich an der radial außen liegenden Seite der gesamte Bereich der Abflachung für diese Angaben berücksichtigt wird, einschließlich des Abschnitts, in dem die Außenkontur des Querschnitts zu beiden Seiten der Rille eine Gerade aufweist. Deutlich erkennbar ist auch, dass die Rille, die im Querschnitt eine einseitig offene runde Form hat, zentral innerhalb der durch eine Gerade definierte Abflachung liegt, und die Abschnitte der durch die Gerade definierten Abflachung zu beiden Seiten der Rille gleich lang sind.

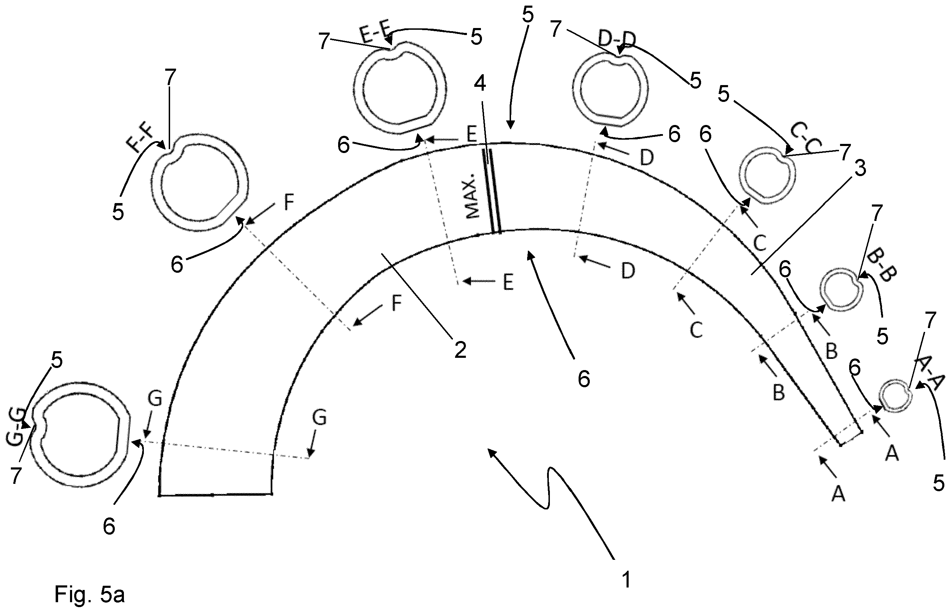

Figur 5a zeigt einen Dilatator 1, dessen Grundform, ungeachtet von Abflachungen an der radial außen liegenden Seite 5 bzw. radial innen liegenden Seite 6, von den Grundformen in den Figuren 4 abweicht. Bei dieser Ausführungsform sind sowohl der Griffbereich 2 als auch ein Teilabschnitt des Dilatationsbereichs 3 gebogen, wobei der Teilabschnitt des Dilatationsbereichs, der eine Biegung aufweist, sich von der Markierung 4, an der der Griffbereich 2 in den Dilatationsbereich 3 übergeht, bis zu der axialen Position des Querschnitts B-B erstreckt. Der von dem Griffbereich 2 abgewandte Endabschnitt des Dilatationsbereichs 3, von der axialen Position des Querschnitts B-B an bis zum distalen Ende, verläuft vollständig gerade.

Wie in den Figuren 1 bis 4 setzt sich die Erweiterung des Dilatationsbereichs 3 in den Griffbereich 2 fort, der ebenfalls sich erweiternd ausgestaltet ist.

Der Dilatator 1 weist sowohl an seiner radial innen liegenden Seite 5, als auch an der radial außen liegenden Seite 6 eine Abflachung auf, wobei sich der Unterabschnitt, in dem sich die Abflachung an der radial außen liegenden Seite 5 und radial innen liegenden Seite 6 erstreckt, über den gesamten Dilatationsbereich erstreckt, d.h. den Teilabschnitt, der eine Biegung aufweist, sowie den geraden Endabschnitt, die oben beschrieben wurden.

Die Abflachung an der radial außen liegenden Seite 5 ist, wie bei der Ausführungsform in Figur 2, durch eine Rille 7 definiert, während die Abflachung an der radial innen liegenden Seite 6 durch eine Gerade definiert wird. In dem gesamten Dilatationsbereich weist der Dilatator an der jeweiligen axialen Position, an einem Querschnitt im gesamten Bereich des Umfangs eine gleichstarke Rohrwandung auf, d.h. die Abflachungen entsprechen im Wesentlichen einer Verformung und nicht einer Verringerung der Wandstärke des Dilatators an der jeweiligen axialen Position. Ungeachtet dessen wird aus den Querschnitten G-G bis A-A deutlich, dass sich die Stärke der Rohrwandung vom Ende des Griffbereichs 2 des Dilatators bis zum distalen Ende des Dilatators im Wesentlichen kontinuierlich verringert.

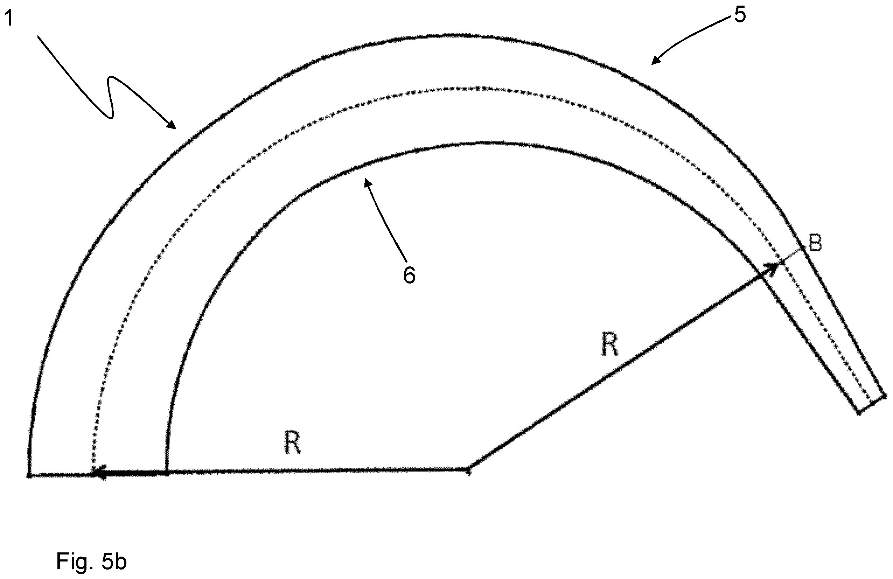

In Figur 5b wird die Biegung des Dilatators von dem Griffbereich bis zu der axialen Position bei dem Querschnitt B-B in einem Längsschnitt des Dilatators verdeutlicht. Dabei verläuft eine gedachte zentrale Linie innerhalb des Dilatators, die gestrichelt dargestellt ist, wobei die zentrale Linie zentral innerhalb eines imaginären zur runden Form ergänzten Dilatators verläuft, d.h. bei der Position der Linie werden die Abflachungen an der radial außen liegenden Seite und gegebenenfalls radial innen liegenden Seite nicht berücksichtigt. Anhand der als Pfeile dargestellten Radien R wird deutlich, dass bei der in Figur 5 gezeigten Ausführungsform der Radius der Biegung, die sich in diesem Fall nicht nur innerhalb des Dilatationsbereichs 3, sondern auch über den Griffbereich 2 erstreckt, konstant bleibt, wobei ein Endabschnitt am distalen Ende gerade verläuft.

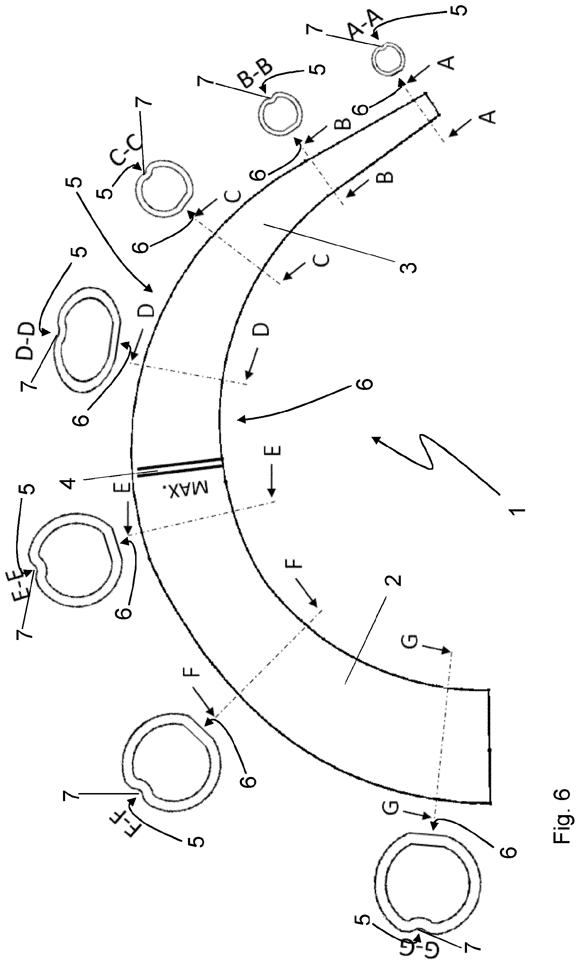

Die in Figur 6 dargestellte Ausführungsform entspricht im Wesentlichen der in Figur 5 gezeigten. Diese Ausführungsform weist jedoch im Bereich der axialen Position des Querschnitts D-D einen weiteren Unterabschnitt mit einer stärker ausgeprägten Abflachungen an der radial außen liegenden 5 und radial innen liegenden Seite 6 auf, bei dem zudem im Vergleich zu den in den anderen Bereichen des Dilatationsbereichs 3 bestehenden Abflachungen die Außenkontur des Querschnitts des Dilatators eine Krümmung mit einem größeren Krümmungsradius aufweist als der Krümmungsradius eines minimalen Kreises, in den die Außenkontur einbeschrieben werden kann. Die Abflachung an der radial außen liegenden Seite 5 wird in diesem weiteren Unterabschnitt sowohl durch einen größeren Krümmungsradius als auch durch eine Gerade und die Rille 7 definiert, während die Abflachung an der radial innen liegenden Seite 6 durch einen größeren Krümmungsradius und durch eine Gerade definiert wird. Im Bereich der axialen Position des Querschnitts D-D ist bei dieser Ausführungsform der Durchmesser senkrecht zu der Richtung von radial innen liegender zu radial außen liegender Seite größer als der maximale Durchmesser an der axialen Position der Markierung 4, der dort 17,3 mm beträgt.

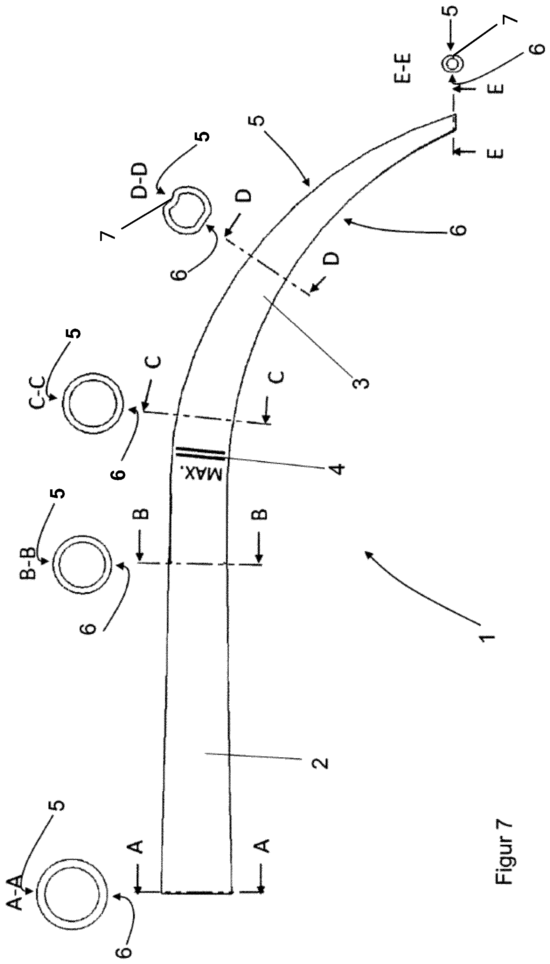

Die in Figur 7 dargestellte Ausführungsform entspricht im Wesentlichen der in Figur 4 dargestellten Ausführungsform, weicht jedoch darin von dieser ab, dass sich der eine Unterabschnitt des Dilatationsbereiches 3 nicht vollständig über den gesamten Dilatationsbereich 3 erstreckt, sondern lediglich von dessen distalem Ende bis zu einer axialen Position, die sich zwischen den Positionen der Querschnitte D-D und C-C befindet. Zwischen den axialen Positionen an den Querschnitten D-D und C-C findet sich ein Übergangsbereich, in dem der Außenquerschnitt des Dilatators von dem Unterabschnitt, der sowohl auf der radial außenliegenden Seite als auch auf der radial innenliegenden Seite eine Abflachung aufweist, konstant zu einem runden Außenquerschnitt an der axialen Position des Querschnitts C-C übergeht. In dem proximalen Abschnitt des Dilatationsbereiches 3, an den der Griffbereich 2 angrenzt, ist demnach keine Abflachung vorhanden.

Im Übrigen entspricht die Abflachung in dem Unterabschnitt des Dilatationsbereichs 3, der sich über die axiale Position des Querschnitts D-D zur axialen Position des Querschnitts E-E erstreckt, in ihrer Ausgestaltung der für Figur 4 beschriebenen Abflachung in diesem Bereich.

## Patentansprüche

1. Dilatator (1) zum Erweitern eines Tracheostomas, der rohrförmig ist und einen Griffbereich (2) und einen sich erweiternden Dilatationsbereich (3) aufweist, wobei zumindest ein Teilabschnitt des Dilatationsbereichs (3) eine Biegung aufweist, so dass der Dilatator (1) eine radial außen (5) und eine radial innen (6) liegenden Seite aufweist, **dadurch gekennzeichnet, dass** in wenigstens einem Unterabschnitt des Dilatationsbereichs (3) der Außenquerschnitt des Dilatators (1) im Vergleich zu einem runden Querschnitt wenigstens an der radial außen liegenden Seite (5) des Dilatators (1) abgeflacht ist, wobei der Dilatator (1) in wenigstens einem Unterabschnitt des Dilatationsbereichs (3) an der radial außen liegenden Seite (5) des Dilatators (1) eine Rille (7) aufweist, die in Längsrichtung des Dilatators (1) verläuft, und in dem Unterabschnitt, in dem die Rille (7) an der radial außen liegenden Seite (5) des Dilatators (1) in Längsrichtung verläuft, die Abflachung im Außenquerschnitt durch eine Gerade definiert ist und die Rille (7) zentral innerhalb der Abflachung verläuft.

2. Dilatator (1) gemäß Anspruch 1, **dadurch gekennzeichnet, dass** die Außenkontur des Querschnitts des Dilatators (1) in wenigstens einem Unterabschnitt des Dilatationsbereichs (3), in dem der Außenquerschnitt des Dilatators (1) im Vergleich zu einem runden Querschnitt wenigstens an der radial außen liegenden Seite (5) des Dilatators (1) abgeflacht ist, zusätzlich an der radial innen liegenden Seite (6) des Dilatators (1) abgeflacht ist.

3. Dilatator (1) gemäß einem der vorangehenden Ansprüche, **dadurch gekennzeichnet, dass** die Außenkontur des Querschnitts des Dilatators (1) in dem wenigstens einen Unterabschnitt in einem Kreissektor des Querschnitts abgeflacht ist, der sich über einen Winkelbereich von 20° bis 100°, vorzugsweise 30° bis 60° erstreckt, bezogen auf einen minimalen Kreis, in den die Außenkontur des Querschnitts an der jeweiligen axialen Position einbeschrieben werden kann.

4. Dilatator (1) gemäß einem der vorangehenden Ansprüche, **dadurch gekennzeichnet, dass** in dem wenigstens einen Unterabschnitt ein Querschnittsdurchmesser der Außenkontur, gemessen von der radial innen liegenden zur der radial außen liegenden Seite (5) des Dilatators (1), um 3,5 % bis 30 %, vorzugsweise 5 % bis 20 %, geringer ist als ein Querschnittsdurchmesser senkrecht dazu.

5. Dilatator (1) gemäß einem der vorangehenden Ansprüche, **dadurch gekennzeichnet, dass** die Rille (7) eine Tiefe im Bereich von 0,05 mm bis 2,5 mm aufweist, vorzugsweise im Bereich von 0,3 mm bis 1,5 mm.

6. Dilatator (1) gemäß einem der vorangehenden Ansprüche, **dadurch gekennzeichnet, dass** die Breite der Rille (7) im Bereich von 1 mm bis 10 mm beträgt und/oder dass die Breite proportional zum Querschnittsdurchmesser der Außenkontur des Dilatators (1), gemessen senkrecht zu der Richtung von radial außen liegender zu radial innen liegender Seite, zunimmt.

7. Dilatator (1) gemäß einem der vorangehenden Ansprüche, **dadurch gekennzeichnet, dass** in dem sich vom distalen Ende zum Griff (2) hin erweiternden Dilatationsbereich (3) der äußere Umfang des Dilatators (1) kontinuierlich zunimmt.

8. Dilatator (1) gemäß einem der vorangehenden Ansprüche, **dadurch gekennzeichnet, dass** der Dilatationsbereich (3) mehrere Unterabschnitte aufweist, in denen der Außenquerschnitts des Dilatators (1) im Vergleich zu einem runden Querschnitt wenigstens an der radial außen liegenden Seite (5) des Dilatators (1) abgeflacht ist, wobei sich wenigstens ein Unterabschnitt der mehreren Unterabschnitten von den jeweils anderen Unterabschnitte des Dilatators (1) dadurch unterscheidet, dass die Abflachung des Außenquerschnitts eine andere Form, Ausdehnung und/ oder Ausprägung aufweist.

9. Dilatator (1) gemäß einem der vorangehenden Ansprüche, **dadurch gekennzeichnet, dass** sich der wenigstens eine Unterabschnitt, in dem der Außenquerschnitts des Dilatators (1) im Vergleich zu einem runden Querschnitt wenigstens an der radial außen liegenden Seite (5) des Dilatators (1) abgeflacht ist, über den gesamten Dilatationsbereich (3) erstreckt.

10. Dilatator (1) gemäß einem der vorangehenden Ansprüche, **dadurch gekennzeichnet, dass** der Dilatator (1) unabhängig von der Auftrennung in Griffbereich (2) und Dilatationsbereich (3) über wenigstens 80 % seiner Längserstreckung, vorzugsweise wenigstens 90 % und bis zu 100 % seiner Längserstreckung gebogen ist, wobei er vorzugsweise an jeder gebogenen axialen Position den gleichen Biegungsradius aufweist.

11. Dilatator (1) gemäß einem der vorangehenden Ansprüche, **dadurch gekennzeichnet, dass** der rohrförmige Dilatator (1) in seinem Lumen einen rohrförmigen Führungskatheter zur Aufnahme eines Führungsdrahtes aufweist.

12. Tracheostomiekanüle mit einem Dilatator (1) gemäß einem der vorangehenden Ansprüche.

13. Tracheostomiekanüle mit einem Dilatator (1) gemäß Anspruch 12, **dadurch gekennzeichnet, dass** die äußere Form der Tracheostomiekanüle mit der inneren Form des Dilatators (1) korrespondiert.

## Claims

1. A dilator (1) for dilating a tracheostoma which is tubular and has a gripping region (2) and an enlarging dilation region (3), wherein at least a portion of the dilation region (3) has a bend so that the dilator (1) has a radially outward (5) and a radially inward (6) side, **characterised in that** in at least a subportion of the dilation region (3) the outside cross-section of the dilator (1) is flattened in comparison with a round cross-section at least at the radially outward side (5) of the dilator (1), wherein the dilator (1) in at least a subportion of the dilation region (3) at the radially outward side (5) of the dilator (1) has a groove (7) extending in the longitudinal direction of the dilator (1) and in the subportion in which the groove (7) extends in the longitudinal direction at the radially outward side (5) of the dilator (1) the flattening in the outside cross-section is defined by a straight line and the groove (7) extends centrally within the flattening.

2. A dilator (1) according to claim 1 **characterised in that** the outside contour of the cross-section of the dilator (1) in at least a subportion of the dilation region (3) in which the outside cross-section of the dilator (1) is flattened in comparison with a round cross-section at least at the radially outward side (5) of the dilator (1) is additionally flattened at the radially inward side (6) of the dilator (1).

3. A dilator (1) according to one of the preceding claims **characterised in that** the outside contour of the cross-section of the dilator (1) in the at least one subportion is flattened in a circular sector of the cross-section, which extends over an angular region of 20° to 100°, preferably 30° to 60°, with respect to a minimum circle in which the outside contour of the cross-section can be inscribed at the respective axial position.

4. A dilator (1) according to one of the preceding claims **characterised in that** in the at least one subportion a cross-sectional diameter of the outside contour, measured from the radially inward to the radially outward side (5) of the dilator (1), is less by 3.5% to 30%, preferably 5% to 20%, than a cross-sectional diameter perpendicular thereto.

5. A dilator (1) according to one of the preceding claims **characterised in that** the groove (7) is of a depth in the region of 0.05 mm to 2.5 mm, preferably in the region of 0.3 mm to 1.5 mm.

6. A dilator (1) according to one of the preceding claims **characterised in that** the width of the groove (7) is in the region of 1 mm to 10 mm and/or the width increases proportionally to the cross-sectional diameter of the outside contour of the dilator (1) measured perpendicularly to the direction from the radially outward to the radially inward side.

7. A dilator (1) according to one of the preceding claims **characterised in that** the outer periphery of the dilator (1) continuously increases in the dilation region (3) which enlarges from the distal end towards the gripping region (2).

8. A dilator (1) according to one of the preceding claims **characterised in that** the dilation region (3) has a plurality of subportions in which the outside cross-section of the dilator (1) is flattened in comparison with a round cross-section at least at the radially outward side (5) of the dilator (1), wherein at least one subportion of the plurality of subportions differs from the respective other subportions of the dilator (1) **in that** the flattening of the outside cross-section is of a different shape, and/or markedness.

9. A dilator (1) according to one of the preceding claims **characterised in that** the at least one subportion in which the outside cross-section of the dilator (1) is flattened in comparison with a round cross-section at least at the radially outward side (5) of the dilator (1) extends over the entire dilation region (3).

10. A dilator (1) according to one of the preceding claims **characterised in that** the dilator (1) is bent independently of the separation into the gripping region (2) and the dilation region (3) over at least 80% of its longitudinal extent, preferably at least 90% and up to 100% of its longitudinal extent, wherein it preferably has the same bending radius at each bent axial position.

11. A dilator (1) according to one of the preceding claims **characterised in that** the tubular dilator (1) in its lumen has a tubular guide catheter for receiving a guide wire.

12. A tracheostomy cannula having a dilator (1) according to one of the preceding claims.

13. A tracheostomy cannula having a dilator (1) according to claim 12 **characterised in that** the external shape of the tracheostomy cannula corresponds to the internal shape of the dilator (1).

## Revendications

1. Dilatateur (1) pour élargissement d'un trachéostome, qui est tubulaire et présente une zone de préhension (2) et une zone de dilatation (3) s'élargissant, dans lequel au moins une section partielle de la zone de dilatation (3) présente une courbure, de sorte que le dilatateur (1) présente un côté situé radialement à l'extérieur (5) et un côté situé radialement à l'intérieur (6), **caractérisé en ce que** dans au moins une sous-section de la zone de dilatation (3), la section transversale extérieure du dilatateur (1) est aplatie par rapport à une section transversale arrondie au moins au niveau du côté situé radialement à l'extérieur (5) du dilatateur (1), dans lequel le dilatateur (1) présente dans au moins une sous-section de la zone de dilatation (3), au niveau du côté situé radialement à l'extérieur (5) du dilatateur (1), une rainure (7) qui s'étend dans la direction longitudinale du dilatateur (1), et dans la sous-section dans laquelle la rainure (7) s'étend au niveau du côté situé radialement à l'extérieur (5) du dilatateur (1) dans la direction longitudinale, l'aplatissement est défini dans la section transversale extérieure par une droite et la rainure (7) s'étend centralement à l'intérieur de l'aplatissement.

2. Dilatateur (1) selon la revendication 1, **caractérisé en ce que** le contour extérieur de la section transversale du dilatateur (1), dans au moins une sous-section de la zone de dilatation (3) dans laquelle la section transversale extérieure du dilatateur (1) est aplatie par rapport à une section transversale arrondie au moins au niveau du côté situé radialement à l'extérieur (5) du dilatateur (1), est aplatie en plus au niveau du côté situé radialement à l'intérieur (6) du dilatateur (1).

3. Dilatateur (1) selon l'une des revendications précédentes, **caractérisé en ce que** le contour extérieur de la section transversale du dilatateur (1) est aplati dans la sous-section, au moins au nombre de une, dans un arc de cercle de la section transversale, qui s'étend sur une plage angulaire de 20 à 100°, de préférence de 30° à 60°, par rapport à un cercle minimum dans lequel le contour extérieur de la section transversale peut s'inscrire au niveau de la position axiale respective.

4. Dilatateur (1) selon l'une des revendications précédentes, **caractérisé en ce que** dans la sous-section, au moins au nombre de une, un diamètre de section transversale du contour extérieur, mesuré du côté situé radialement à l'intérieur vers le côté situé radialement à l'extérieur (5) du dilatateur (1), est inférieur de 3,5 % à 30 %, de préférence 5 % à 20 %, à un diamètre de section transversale perpendiculaire à celui-ci.

5. Dilatateur (1) selon l'une des revendications précédentes, **caractérisé en ce que** la rainure (7) présente une profondeur dans la plage de 0,05 mm à 2,5 mm, de préférence dans la plage de 0,3 mm à 1,5 mm.

6. Dilatateur (1) selon l'une des revendications précédentes, **caractérisé en ce que** la largeur de la rainure (7) se trouve dans la plage de 1 mm à 10 mm et/ou que la largeur augmente proportionnellement au diamètre de section transversale du contour extérieur du dilatateur (1), mesuré perpendiculairement à la direction du côté situé radialement à l'extérieur vers le côté situé radialement à l'intérieur.

7. Dilatateur (1) selon l'une des revendications précédentes, **caractérisé en ce que** la circonférence extérieure du dilatateur (1) augmente en continu dans la zone de dilatation (3) s'élargissant de l'extrémité distale vers la poignée (2).

8. Dilatateur (1) selon l'une des revendications précédentes, **caractérisé en ce que** la zone de dilatation (3) présente plusieurs sous-sections dans lesquelles la section transversale extérieure du dilatateur (1) est aplatie par rapport à une section transversale arrondie au moins au niveau du côté situé radialement à l'extérieur (5) du dilatateur (1), au moins une sous-section des plusieurs sous-sections se distinguant des autres sous-sections respectives du dilatateur (1) par le fait que l'aplatissement de la section transversale extérieure présente une autre forme, extension et/ou expression.

9. Dilatateur (1) selon l'une des revendications précédentes, **caractérisé en ce que** la sous-section, au moins au nombre de une, dans laquelle la section transversale extérieure du dilatateur (1) est aplatie par rapport à une section transversale arrondie au moins au niveau du côté situé radialement à l'extérieur (5) du dilatateur (1), s'étend sur toute la zone de dilatation (3).

10. Dilatateur (1) selon l'une des revendications précédentes, **caractérisé en ce que** le dilatateur (1) est incurvé, indépendamment de la séparation dans la zone de préhension (2) et la zone de dilatation (3), sur au moins 80 % de son extension longitudinale, de préférence au moins 90 % et jusqu'à 100 % de son extension longitudinale, celui-ci présentant de préférence le même rayon de courbure au niveau de chaque position axiale incurvée.

11. Dilatateur (1) selon l'une des revendications précédentes, **caractérisé en ce que** le dilatateur tubulaire (1) présente dans son lumen un cathéter de guidage tubulaire destiné à recevoir un fil de guidage.

12. Canule de trachéostomie avec dilatateur (1) selon l'une des revendications précédentes.

13. Canule de trachéostomie avec dilatateur (1) selon la revendication 12, **caractérisée en ce que** la forme extérieure de la canule de trachéostomie correspond à la forme intérieure du dilatateur (1).
